# EUROPEAN PATENT APPLICATION

(11) **EP 2 000 463 A2**
(43) Date of publication of application: **10.12.2008**
(21) Application number: 07739502.8
(22) Date of filing: 23.03.2007
(51) Int. Cl.: C07D 233/58, C07D 401/14, C09K 11/06, H01L 51/50

(54) **NITROGEN-CONTAINING HETEROCYCLIC DERIVATIVE AND ORGANIC ELECTROLUMINESCENT DEVICE USING SAME**

(30) Priority: 27.03.2006 JP 2006086026
(71) Applicant: Idemitsu Kosan Co., Ltd., Chiyoda-ku Tokyo 100-8321 (JP)
(72) Inventor: YAMAMOTO, Hiroshi, Sodegaura-shi, Chiba 299-0293 (JP); ARAKANE, Takashi, Sodegaura-shi, Chiba 299-0293 (JP)
(74) Representative: Gille Hrabal Struck Neidlein Prop Roos
(86) International application number: PCT/JP2007/056061
(87) International publication number: WO 2007/111263

(57) **Abstract**

Provided are a novel nitrogen-containing heterocyclic derivative having a specific structure and an organic electroluminescence device having an organic thin film layer composed of one or a plurality of layers including at least a light emitting layer and interposed between a cathode and an anode, in which at least one layer of the organic thin film layer contains the nitrogen-containing heterocyclic derivative alone or as a component of a mixture. With this, the organic electroluminescence device capable of being driven at a low voltage and having high emission luminance and high luminous efficiency can be realized.

## Description

### TECHNICAL FIELD

The present invention relates to a novel nitrogen-containing heterocyclic derivative having a specific substituent, and a material for an organic electroluminescence (EL) device and an organic EL device each using the derivative, in particular, an organic EL device having high luminous efficiency and a long lifetime by using a nitrogen-containing heterocyclic derivative useful as a component of an organic EL device in at least one layer of its organic thin film layer.

### BACKGROUND ART

A large number of organic electroluminescence (EL) devices each using an organic substance have been developed because of their potential to find applications in solid emission type, inexpensive, large-area, full-color display devices. In general, an EL device is composed of a light emitting layer and a pair of opposing electrodes between which the layer is interposed. Light emission is the following phenomenon: upon application of an electric field between both electrodes, an electron is injected from a cathode side and a hole is injected from an anode side, and furthermore, the electron recombines with the hole in the light emitting layer to produce an excited state, and energy generated upon return to a ground state from the excited state is radiated as light.
A conventional organic EL device is driven at a voltage higher than the voltage at which an inorganic light emitting diode is driven, and has lower emission luminance and lower luminous efficiency than those of the inorganic light emitting diode. In addition, the properties of the device deteriorate so remarkably that the device cannot be put into practical use. Although a recent organic EL device has been gradually improved, an additionally low voltage at which the device is driven, additionally high emission luminance, and additionally high luminous efficiency have been requested of the device.
To solve those problems, for example, Patent Document 1 discloses a device using a compound having a benzimidazole structure as a light emitting material, and describes that the device emits light with a luminance of 200 cd/m² at a voltage of 9 V. In addition, Patent Document 2 describes a compound having a benzimidazole ring and an anthracene skeleton. Patent Document 3 describes a compound having an imidazole ring, the compound being used in a light emitting layer or in a hole blocking layer. However, there has been a demand for a device which: has higher emission luminance and higher luminous efficiency than those of an organic EL device using any one of those compounds; and can be driven at a voltage lower than the voltage at which the organic EL device is driven.

Patent Document 1:US-A-5,645,948
Patent Document 2: JP-A-2002-38141
Patent Document 3: JP-A-2004-146368

### DISCLOSURE OF THE INVENTION

### PROBLEMS TO BE SOLVED BY THE INVENTION

The present invention has beenmade with a view to solving the above-mentioned problems, and an object of the present invention is to provide a novel nitrogen-containing heterocyclic derivative useful as a component of an organic EL device to realize an organic EL device capable of being driven at a low voltage and having high emission luminance and high luminous efficiency by using the nitrogen-containing heterocyclic derivative in at least one layer of its organic thin film layer.

### MEANS FOR SOLVING THE PROBLEMS

The inventors of the present invention have made extensive studies with a view to achieving the above-mentioned obj ect. As a result, they have found that with the use of a novel nitrogen-containing heterocyclic derivative having a specific structure in at least one layer of the organic thin film layer of an organic EL device, a reduction in voltage at which the organic EL device is driven, and improvements in luminance and efficiency of the device can be achieved. Thus, they have completed the present invention.

That is, according to the present invention, there is provided a nitrogen-containing heterocyclic derivative represented by the following general formula (1): 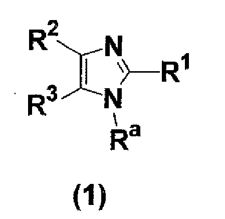 in the general formula (1): R¹ to R³ each independently represent a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted by a substituted or unsubstituted aryl group having 6 to 60 ring carbon atoms, a halogen atom, a cyano group, a nitro group, a hydroxyl group, or a carboxyl group;
R^{a} represents a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, or a substituted or unsubstituted aralkyl group having 6 to 50 ring atoms; and
at least one of R¹ to R³ and R^{a} represents a substituent represented by the following general formula (2):

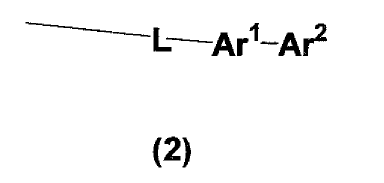

where: L represents a single bond, a substituted or unsubstituted arylene group having 6 to 60 ring carbon atoms, a substituted or unsubstituted heteroarylene group having 5 to 60 ring atoms, or a substituted or unsubstituted fluorenylene group;
Ar¹ represents a substituted or unsubstituted arylene group having 6 to 60 ring carbon atoms, a substituted or unsubstituted heteroarylene group having 5 to 60 ring atoms, or a substituted or unsubstituted fluorenylene group; and
Ar² represents a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted by a substituted or unsubstituted aryl group having 6 to 60 ring carbon atoms, a halogen atom, a cyano group, a nitro group, a hydroxyl group, or a carboxyl group.

The nitrogen-containing heterocyclic derivative according to the present invention is preferably used as a material for an organic EL device.
The organic EL device according to the present invention includes an organic thin film layer composed of one or more layers including at least a light emitting layer and interposed between a cathode and an anode, and at least one layer of the organic thin film layer contains the nitrogen-containing heterocyclic derivative according to the present invention alone or as a component of a mixture.

### EFFECT OF THE INVENTION

An organic EL device using the nitrogen-containing heterocyclic derivative of the present invention can be driven at a low voltage, has high luminous efficiency, and is excellent in electron transporting property.

### BEST MODE FOR CARRYING OUT THE INVENTION

According to the present invention, there is provided a nitrogen-containing heterocyclic derivative represented by the following general formula (1): 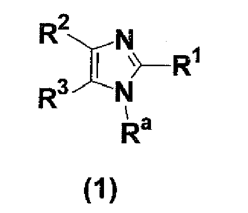 in the general formula (1): R¹ to R³ each independently represent a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted by a substituted or unsubstituted aryl group having 6 to 60 ring carbon atoms, a halogen atom, a cyano group, a nitro group, a hydroxyl group, or a carboxyl group;
R^{a} represents a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, or a substituted or unsubstituted aralkyl group having 6 to 50 ring atoms; and
at least one of R¹ to R³ and R^{a} represents a substituent represented by the following general formula (2):

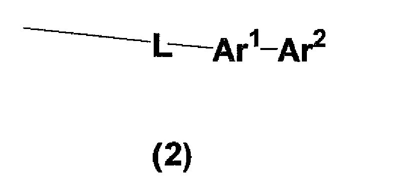

in the general formula (2) : L represents a single bond, a substituted or unsubstituted arylene group having 6 to 60 ring carbon atoms, a substituted or unsubstituted heteroarylene group having 5 to 60 ring atoms, or a substituted or unsubstituted fluorenylene group;
Ar¹ represents a substituted or unsubstituted arylene group having 6 to 60 ring carbon atoms, a substituted or unsubstituted heteroarylene group having 5 to 60 ring atoms, or a substituted or unsubstituted fluorenylene group; and
Ar² represents a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted by a substituted or unsubstituted aryl group having 6 to 60 ring carbon atoms, a halogen atom, a cyano group, a nitro group, a hydroxyl group, or a carboxyl group.

The nitrogen-containing heterocyclic derivative represented by the general formula (1) is preferably a compound represented by the following general formula (1-a), (1-b), or (1-c) : 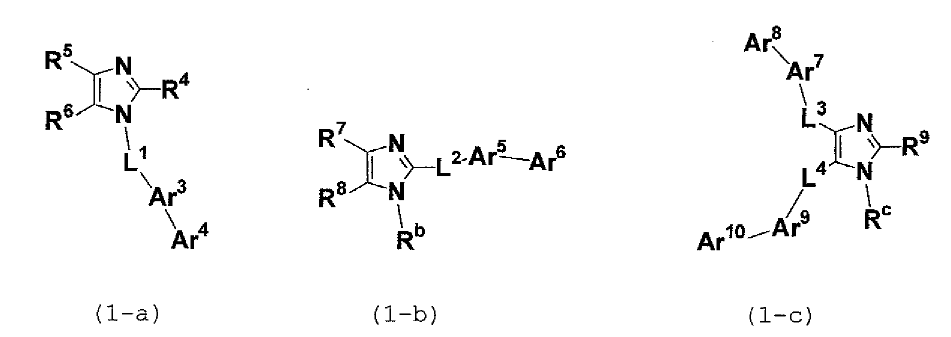 where R⁴ to R⁹ each independently have the same meaning as that of any one of R¹ to R³ in the general formula (1), Ar³, Ar⁵, Ar⁷, and Ar⁹ each independently have the same meaning as that of Ar¹ in the general formula (1), Ar⁴, Ar⁶, Ar⁸, and Ar¹⁰ each independently have the same meaning as that of Ar² in the general formula (1), R^{b} and R^{c} each independently have the same meaning as that of R^{a} in the general formula (1), and L¹, L², L³, and L⁴ each independently have the same meaning as that of L in the general formula (1).

The nitrogen-containing heterocyclic derivative represented by the general formula (1) is preferably a compound represented by the following general formula (1-d) or (1-e): 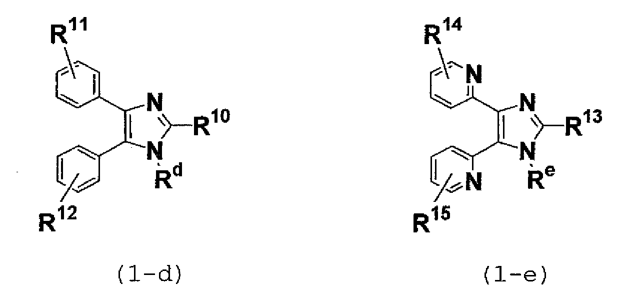 where: R¹⁰ to R¹⁵ each independently have the same meaning as that of R¹ to R³ in the general formula (1);
R^{d} and R^{e} each independently have the same meaning as that of R^{a} in the general formula (1); and
at least one of R¹⁰, R¹³, R^{d}, and R^{e} is a substituent represented by the general formula (2).

Examples of the substituted or unsubstituted aryl group having 6 to 60 ring atoms and the substituted or unsubstituted heteroaryl group having 5 to 60 ring atoms of R¹ to R¹⁵, R^{a} to R^{e}, Ar², Ar⁴, Ar⁶, Ar⁸, and Ar¹⁰ include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl-4-yl group, a p-terphenyl-3-yl group, a p-terphenyl-2-yl group, an m-terphenyl-4-yl group, an m-terphenyl-3-ylgroup, an m-terphenyl-2-yl group,an o-tolyl group, an m-tolyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, a 4"-t-butyl-p-terphenyl-4-yl group, a fluoranthenyl group, a fluorenyl group, a 1-pyrrolyl group, a 2-pyrrolyl group, a 3-pyrrolyl group, a pyrazinyl group, a 2-pyridinyl group, a 3-pyridinyl group, a 4-pyridinyl group, a 1-indolyl group, a 2-indolyl group, a 3-indolyl group, a 4-indolyl group, a 5-indolyl group, a 6-indolyl group, a 7-indolyl group, a 1-isoindolyl group, a 2-isoindolyl group, a 3-isoindolyl group, a 4-isoindolyl group, a 5-isoindolyl group, a 6-isoindolyl group, a 7-isoindolyl group, a 2-furyl group, a 3-furyl group, a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 3-isobenzofuranyl group, a 4-isobenzofuranyl group, a 5-isobenzofuranyl group, a 6-isobenzofuranyl group, a 7-isobenzofuranyl group,a quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isoquinolyl group, an 8-isoquinolyl group, a 2-quinoxalinyl group, a 5-quinoxalinyl group, a 6-quinoxalinyl group, a 1-carbazolyl group, a 2-carbazolyl group, a 3-carbazolyl group, a 4-carbazolyl group, a 9-carbazolyl group, a 1-phenanthridinyl group, a 2-phenanthridinyl group, a 3-phenanthridinyl group, a 4-phenanthridinyl group, a 6-phenanthridinyl group, a 7-phenanthridinyl group, an 8-phenanthridinyl group, a 9-phenanthridinyl group, a 10-phenanthridinyl group, a 1-acridinyl group, a 2-acridinyl group, a 3-acridinyl group, a 4-acridinyl group, a 9-acridinyl group, a 1,7-phenanthrolin-2-yl group, a 1,7-phenanthrolin-3-yl group, a 1,7-phenanthrolin-4-yl group, a 1,7-phenanthrolin-5-yl group, a 1,7-phenanthrolin-6-yl group, a 1,7-phenanthrolin-8-yl group, a 1,7-phenanthrolin-9-yl group, a 1,7-phenanthrolin-10-yl group, a 1,8-phenanthrolin-2-yl group, a 1,8-phenanthrolin-3-yl group, a 1,8-phenanthrolin-4-yl group, a 1,8-phenanthrolin-5-yl group, a 1,8-phenanthrolin-6-yl group, a 1,8-phenanthrolin-7-yl group, a 1,8-phenanthrolin-9-yl group, a 1,8-phenanthrolin-10-yl group, a 1,9-phenanthrolin-2-yl group, a 1,9-phenanthrolin-3-yl group, a 1,9-phenanthrolin-4-yl group, a 1,9-phenanthrolin-5-yl group, a 1,9-phenanthrolin-6-yl group, a 1,9-phenanthrolin-7-yl group, a 1,9-phenanthrolin-8-yl group, a 1,9-phenanthrolin-10-yl group, a 1,10-phenanthrolin-2-yl group, a 1,10-phenanthrolin-3-yl group, a 1,10-phenanthrolin-4-yl group, a 1,10-phenanthrolin-5-yl group, a 2,9-phenanthrolin-1-yl group, a 2,9-phenanthrolin-3-yl group, a 2,9-phenanthrolin-4-yl group, a 2,9-phenanthrolin-5-yl group, a 2,9-phenanthrolin-6-yl group, a 2,9-phenanthrolin-7-yl group, a 2,9-phenanthrolin-8-yl group, a 2,9-phenanthrolin-10-yl group, a 2,8-phenanthrolin-1-yl group, a 2,8-phenanthrolin-3-yl group, a 2,8-phenanthrolin-4-yl group, a 2,8-phenanthrolin-5-yl group, a 2,8-phenanthrolin-6-yl group, a 2,8-phenanthrolin-7-yl group, a 2,8-phenanthrolin-9-yl group, a 2,8-phenanthrolin-10-yl group, a 2,7-phenanthrolin-1-yl group, a 2,7-phenanthrolin-3-yl group, a 2,7-phenanthrolin-4-yl group, a 2,7-phenanthrolin-5-yl group, a 2,7-phenanthrolin-6-yl group, a 2,7-phenanthrolin-8-yl group, a 2,7-phenanthrolin-9-yl group, a 2,7-phenanthrolin-10-yl group, a 1-phenazinyl group, a 2-phenazinyl group, a 1-phenothiazinyl group, a 2-phenothiazinyl group, a 3-phenothiazinyl group, a 4-phenothiazinyl group, a 10-phenothiazinyl group, a 1-phenoxazinyl group, a 2-phenoxazinyl group, a 3-phenoxazinyl group, a 4-phenoxazinyl group, a 10-phenoxazinyl group, a 2-oxazolyl group, a 4-oxazolyl group, a 5-oxazolyl group, a 2-oxadiazolyl group, a 5-oxadiazolyl group, a 3-furazanyl group, a 2-thienyl group, a 3-thienyl group, a 2-methylpyrral-2-yl group, a2-methylpyrrol-3-yl group, a 2-methylpyrrol-4-yl group, a 2-methylpyrrol-5-yl group, a 3-methylpyrrol-1-yl group, a 3-methylpyrrol-2-yl group, a 3-methylpyrrol-4-yl group, a 3-methylpyrrol-5-yl group, a 2-t-butylpyrrol-4-yl group, a 3-(2-phenylpropyl)pyrrol-1-yl group, a 2-methyl-1-indolyl group, a 4-methyl-1-indolyl group, a 2-methyl-3-indolyl group, a 4-methyl-3-indolyl group, a 2-t-butyl-1-indolyl group, a 4-t-butyl-1-indolyl group, a 2-t-butyl-3-indolyl group, and a 4-t-butyl-3-indolyl group.
Of those, a phenyl group, a naphthyl group, a biphenyl group, an anthracenyl group, a phenanthryl group, a pyrenyl group, a chrysenyl group, a fluoranthenyl group, and a fluorenyl group are preferable.

Examples of the alkyl group having 1 to 50 carbon atoms of R¹ to R¹⁵, R^{a} to R^{e}, Ar², Ar⁴, Ar⁶, Ar⁸, and Ar¹⁰ include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group,a chloromethyl group,a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1, 2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, and a 1,2,3-trinitropropyl group.

Specific examples of the substituted or unsubstituted cycloalkyl group having 3 to 50 carbon atoms of R¹ to R¹⁵, R^{a} to R^{e}, Ar², Ar⁴, Ar⁶, Ar⁸, and Ar¹⁰ include a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

Examples of the substituted or unsubstituted aralkyl group having 6 to 50 ring atoms of R¹ to R¹⁵, R^{a} to R^{e}, Ar², Ar⁴, Ar⁶, Ar⁸, and Ar¹⁰ include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naph-thylisopropyl group, a β-naphthylmethyl group, a 1-β-naphthylethyl group, a 2-β-naphthylethyl group, a 1-β-naphthylisopropyl group, a 2-β-naphthylsopropyl group, a 1-pyrrolylmethyl group, a 2-(1-pyrrolyl)ethyl group, a p-methylbenzyl group, an m-methylbenzyl group, an o-methylbenzyl group, a p-chlorobenzyl group, an m-chlorobenzyl group, an o-chlorobenzyl group, a p-bromobenzyl group, anm-bromobenzyl group, an o-bromobenzyl group, a p-iodobenzyl group, an m-iodobenzyl group, an o-iodobenzyl group, a p-hydroxybenzyl group, an m-hydroxybenzyl group, an o-hydroxybenzyl group, a p-aminobenzyl group, an m-aminobenzyl group, an o-aminobenzyl group, a p-nitrobenzyl group, an m-nitrobenzyl group, an o-nitrobenzyl group, a p-cyanobenzyl group, an m-cyanobenzyl group, an o-cyanobenzyl group, a 1-hydroxy-2-phenylisopropyl group, and a 1-chloro-2-phenylisopropyl group.

The substituted or unsubstituted alkoxy group having 1 to 50 carbon atoms of R¹ to R¹⁵, Ar², Ar⁴, Ar⁶, Ar⁸, and Ar¹⁰ is represented by -OY, and examples of Y include the same examples as those described for the above-mentioned alkyl group.
The substituted or unsubstituted aryloxy group having 5 to 50 ring atoms of R¹ to R¹⁵, Ar², Ar⁴, Ar⁶, Ar⁸, and Ar¹⁰ is represented by-OY', and examples of Y' include examples similar to those described for the aryl group.
The substituted or unsubstituted arylthio group having 5 to 50 ring atoms of R¹ to R¹⁵, Ar², Ar⁴, Ar⁵, Ar⁸, and Ar¹⁰ is represented by-SY', and examples of Y' include examples similar to those described for the aryl group.
The substituted or unsubstituted alkoxycarbonyl group having 1 to 50 carbon atoms of R¹ to R¹⁵, Ar², Ar⁴, Ar⁶, Ar⁸, and Ar¹⁰ is a group represented by -COOY, and examples of Y include examples similar to those described for the alkyl group.
Examples of a substituted or unsubstituted aryl group in the amino group substituted by the aryl group having 6 to 60 ring atoms of R¹ to R¹⁵, Ar², Ar⁴, Ar⁶, Ar⁸, and Ar¹⁰ include examples similar to those described for the aryl group.
Examples of the halogen atom of R¹ to R¹⁵, Ar², Ar⁴, Ar⁶, Ar⁸, and Ar¹⁰ include a fluorine atom, a chlorine atom, a bromine atom, and an iodine atom.

A specific example of the substituted or unsubstituted arylene group having 6 to 60 ring carbon atoms represented by any one of L, L¹ to L⁴, Ar¹, Ar³, Ar⁵, Ar⁷, and Ar⁹ is a divalent substituent obtained by removing an additional one hydrogen atom from a substituent described for the aryl group, and preferable examples of the divalent substituent include a phenylene group, a naphthylene group, a biphenylene group, an anthranylene group, a phenanthrylene group, a pyrenylene group, a chrysenylene group, a fluoranthenylene group, and a fluorenylene group.
A specific example of the substituted or unsubstituted heteroarylene group having 5 to 60 ring atoms represented by any one of L, L¹ to L⁴, Ar¹, Ar³, Ar⁵, Ar⁷, and Ar⁹ is a divalent substituent obtained by removing an additional one hydrogen atom from a substituent described for the heteroaryl group. Examples of the divalent substituent include divalent groups each obtained by removing a hydrogen atom from a pyridyl group, a pyrazyl group, a quinolyl group, an isoquinolyl group, a phenanthrolyl group, a furyl group, a benzofuryl group, a dibenzofuryl group, a thienyl group, a dibenzothienyl group, a benzothienyl group, a pyrrolyl group, an indolyl group, a carbazolyl group, an imidazolyl group, a benzimidazolyl group, or the like, and preferable examples of the divalent substituent include divalent groups each obtained by removing a hydrogen atom from a pyridyl group, a quinolyl group, a carbazolyl group, or an indolyl group.

Specific examples of the nitrogen-containing heterocyclic derivative represented by the general formula (1) of the present invention are shown below. However, the present invention is not limited to these exemplified compounds.

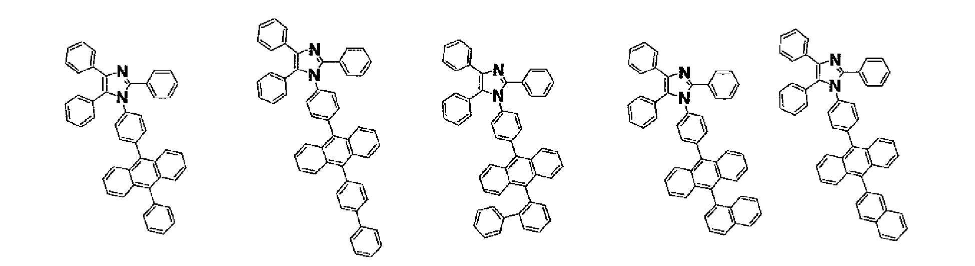

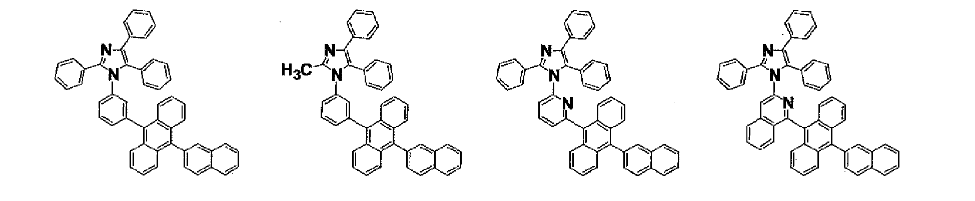

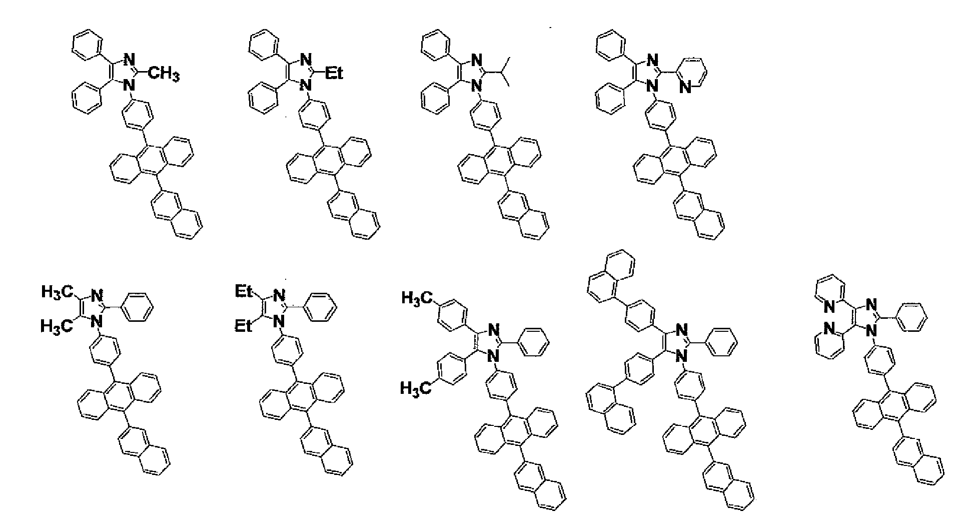

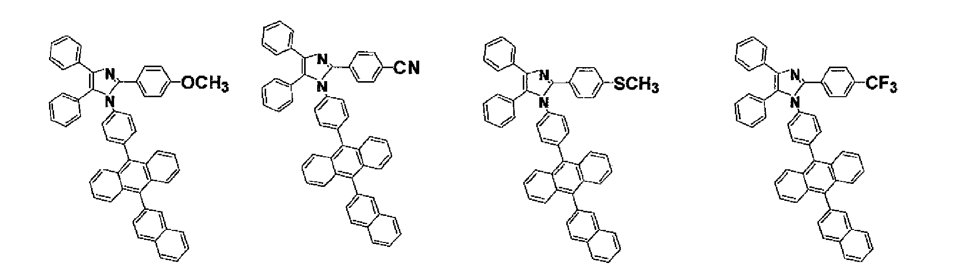

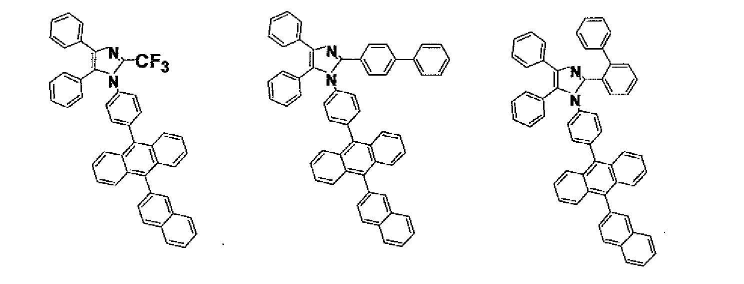

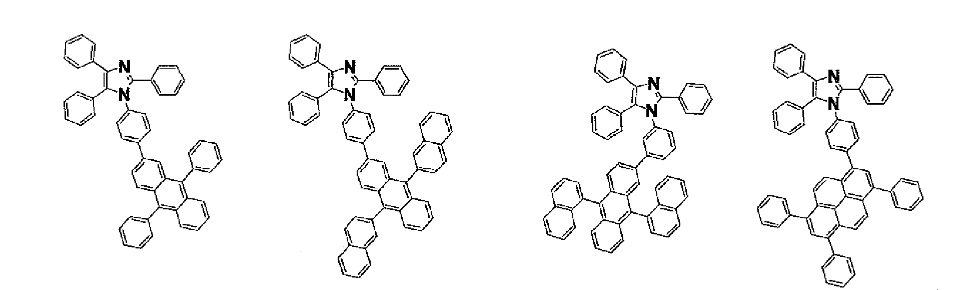

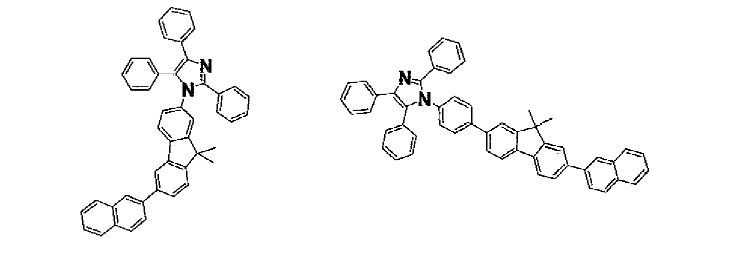

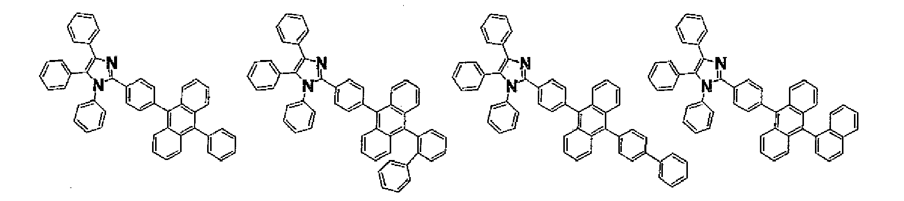

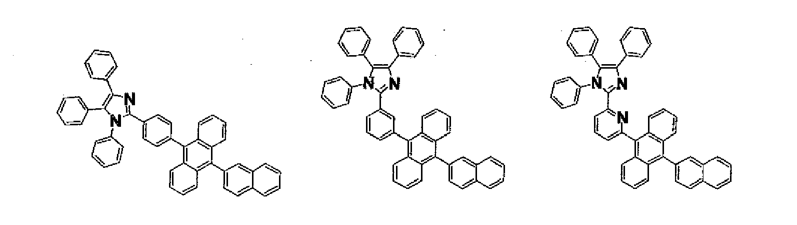

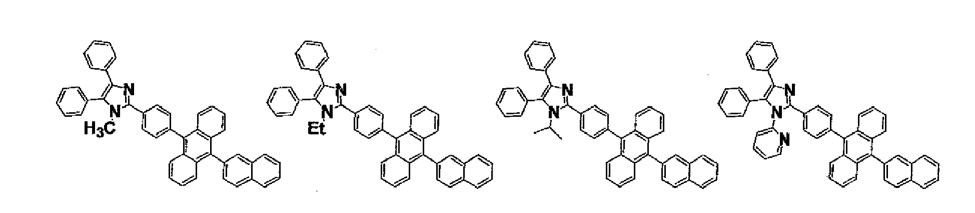

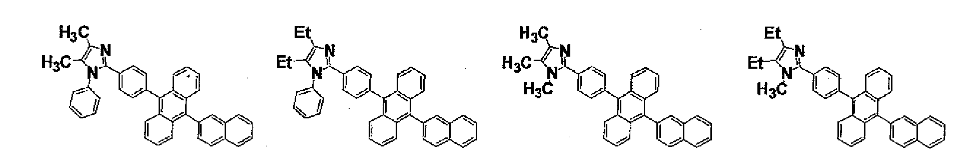

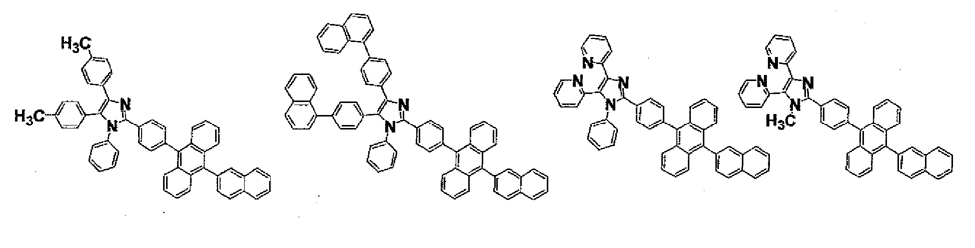

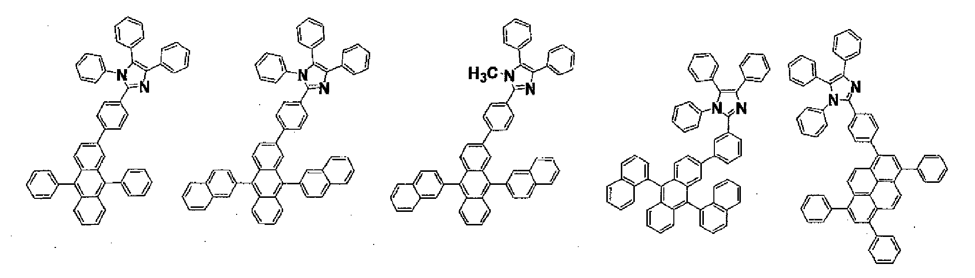

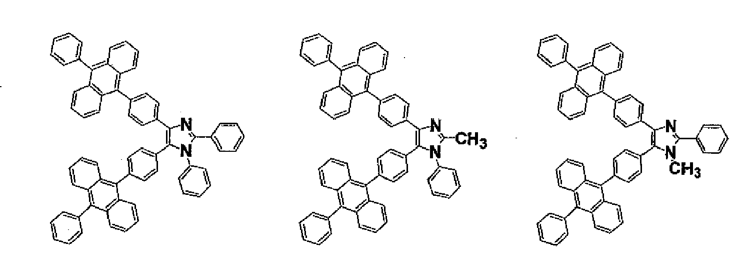

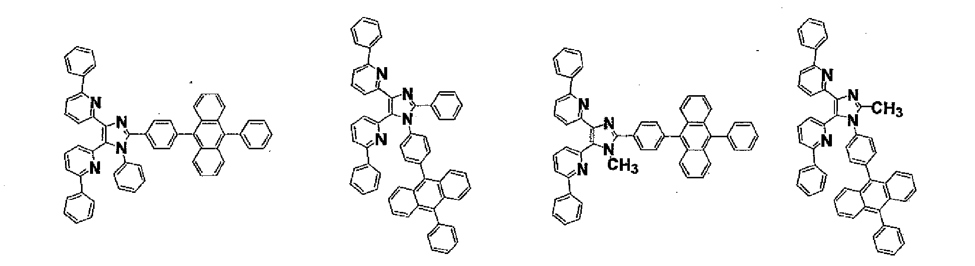

The nitrogen-containing heterocyclic derivative of the present invention is a material for an organic EL device.
The nitrogen-containing heterocyclic derivative of the present invention is preferably an electron injecting material or an electron transporting material for an organic EL device.
The nitrogen-containing heterocyclic derivative of the present invention is preferably a light emitting material for an organic EL device.

Next, the organic EL device of the present invention will be described.
An organic EL device of the present invention includes one or multiple organic thin film layers including at least a light emitting layer, the one or multiple organic thin film layers being interposed between a cathode and an anode, in which at least one layer of the one or more multiple organic thin film layers contains the nitrogen-containing heterocyclic derivative alone or as a component of a mixture.
In the organic EL device of the present invention, it is preferable that: the organic thin film layers described above have an electron injecting layer or an electron transporting layer; and the electron injecting layer or the electron transporting layer contain the nitrogen-containing heterocyclic derivative of the present invention alone or as a component of a mixture.
The organic EL device of the present invention is preferably an organic EL device having an organic thin film layer composed of one or two or more layers including at least a light emitting layer and interposed between a cathode and an anode, in which the light emitting layer contains the nitrogen-containing heterocyclic derivative of the present invention alone or as a component of a mixture.
The organic EL device of the present invention is preferably such that the electron injecting layer or the electron transporting layer containing the nitrogen-containing heterocyclic derivative of the present invention contains a reducing dopant.
The reducing dopant of the organic EL device of the present invention is preferably at least one kind of a substance selected from the group consisting of an alkali metal, an alkaline earth metal, a rare earth metal, an alkali metal oxide, an alkali metal halide, an alkaline earthmetal oxide, an alkaline earthmetal halide, a rare earth metal oxide, a rare earth metal halide, an organic complex of an alkali metal, an organic complex of an alkaline earth metal, and an organic complex of a rare earth metal.

The structure of the organic EL device of the present invention will be described in the following.

### (1) Organic EL device structure

Typical examples of the structure of the organic EL device of the present invention include the following:
(1) an anode/light emitting layer/cathode;
(2) an anode/hole injecting layer/light emitting layer/cathode;
(3) an anode/light emitting layer/electron injecting layer/cathode;
(4) an anode/light emitting layer/electron transporting layer/electron injecting layer/cathode;
(5) an anode/hole injecting layer/light emitting layer/electron injecting layer/cathode;
(6) an anode/hole injecting layer/light emitting layer/electron transporting layer/electron injecting layer/cathode;
(7) an anode/organic semiconductor layer/light emitting layer/cathode;
(8) an anode/organic semiconductor layer/electron barrier layer/light emitting layer/cathode;
(9) an anode/organic semiconductor layer/light emitting layer/adhesion improving layer/cathode;
(10) an anode/hole injecting layer/hole transporting layer/light emitting layer/electron injecting layer/cathode;
(11) an anode/hole injecting layer/hole transporting layer/light emitting layer/electron transporting layer/electron injecting layer/cathode;
(12) an anode/insulating layer/light emitting layer/insulating layer/cathode;
(13) an anode/inorganic semiconductor layer/insulating layer/light emitting layer/insulating layer/cathode;
(14) an anode/organic semiconductor layer/insulating layer/light emitting layer/insulating layer/cathode;
(15) an anode/insulating layer/hole injecting layer/hole transporting layer/light emitting layer/ insulating layer/cathode;
(16) an anode/insulating layer/hole injecting layer/hole transporting layer/light emitting layer/electron injecting layer/cathode; and
(17) an anode/insulating layer/hole injecting layer/hole transporting layer/light emitting layer/electron transporting layer/electron injecting layer/cathode.
   Of those, the structure (10) or (11) is preferably used in ordinary cases. However, the structures are not limited to the foregoing.
   The nitrogen-containing heterocyclic derivative of the present invention may be used in any one of the organic thin film layers of the organic EL device. The derivative can be used preferably in a light emitting zone or an electron transporting zone. The derivative is used particularly preferably in an electron injecting layer, an electron transporting layer, and a light emitting layer.

### (2) Transparent substrate

The organic EL device of the present invention is prepared on a transparent substrate. Here, the transparent substrate is the substrate which supports the organic EL device. It is preferable that the transparent substrate have a transmittance of light of 50% or greater in the visible region of 400 to 700 nm and be flat and smooth.
Examples of the transparent substrate include glass plates and polymer plates. Specific examples of the glass plate include plates made of soda-lime glass, glass containing barium and strontium, lead glass, aluminosilicate glass, borosilicate glass, barium borosilicate glass, and quartz. Specific examples of the polymer plate include plates made of polycarbonate resins, acrylic resins, polyethylene terephthalate, polyether sulfide, and polysulfone.

### ] (3) Anode

The anode in the organic EL device of the present invention has the function of injecting holes into the hole transporting layer or the light emitting layer. It is effective that the anode has a work function of 4.5 eV or greater. Specific examples of the material for the anode used in the present invention include indium tin oxide (ITO) alloys, tin oxide (NESA), indium zinc oxide (IZO), gold, silver, platinum, and copper.
The anode can be prepared by forming a thin film of the electrode material described above in accordance with a process such as the vapor deposition process and the sputtering process.
When the light emitted from the light emitting layer is obtained through the anode, it is preferable that the anode have a transmittance of the emitted light greater than 10%. It is also preferable that the sheet resistivity of the anode be several hundred Ω/□ or smaller. The thickness of the anode is, in general, selected in the range of 10 nm to 1 µm and preferably in the range of 10 to 200 nm although the preferable range may be different depending on the used material.

### (4) Light emitting layer

The light emitting layer in the organic EL device has a combination of the following functions (1) to (3).
(1) The injecting function: the function of injecting holes from the anode or the hole injecting layer and injecting electrons from the cathode or the electron injecting layer when an electric field is applied.
(2) The transporting function: the function of transporting injected charges (i.e., electrons and holes) by the force of the electric field.
(3) The light emitting function: the function of providing the field for recombination of electrons and holes and leading to the emission of light.
   However, the easiness of injection may be different between holes and electrons and the ability of transportation expressed by the mobility may be different between holes and electrons. It is preferable that either one of the charges be transferred.
   For the process of forming the light emitting layer, a known process such as the vapor deposition process, the spin coating process, and the LB process can be used. It is particularly preferable that the light emitting layer be a molecular deposit film. The molecular deposit film is a thin film formed by deposition of a material compound in the gas phase or a film formed by solidification of a material compound in a solution or in the liquid phase. In general, the molecular deposit film can be distinguished from the thin film formed in accordance with the LB process (i.e., molecular accumulation film) based on the differences in aggregation structure and higher order structure and functional differences caused by those structural differences.
   Further, as disclosed in JP-A-57-51781, the light emitting layer can also be formed by dissolving a binder such as a resin and the material compounds into a solvent to prepare a solution, followed by forming a thin film from the prepared solution by the spin coating process or the like.
   In the present invention, when desired, the light emitting layer may include other known light emitting materials other than the light emitting material composed of the nitrogen-containing heterocyclic derivative of the present invention, or a light emitting layer including other known light emitting material may be laminated to the light emitting layer including the light emitting material composed of the nitrogen-containing heterocyclic derivative of the present invention as long as the object of the present invention is not adversely affected.

Examples of a light emitting material or a doping material which can be used in the light emitting layer include, but not limited to, an arylamine compound and/or a styrylamine compound, anthracene, naphthalene, phenanthrene, pyrene, tetracene, coronene, chrysene, fluorescein, perylene, phthaloperylene, naphthaloperylene, perynone, phthaloperynone, naphthaloperynone, diphenylbutadiene, tetraphenylbutadiene, coumarin, oxadiazole, aldazine, bisbenzoxazoline, bisstyryl, pyrazine, cyclopentadiene, quinoline metal complexes, aminoquinoline metal complexes, benzoquinoline metal complexes, imine, diphenylethylene, vinylanthracene, diaminocarbazole, pyrane, thiopyrane, polymethine, merocyanine, imidazole-chelated oxynoid compounds, quinacridone, rubrene, and fluorescent dyes.

In addition, the organic EL device of the present invention is preferably such that the light emitting layer contains an arylamine compound and/or a styrylamine compound.
Examples of the arylamine compound include compounds each represented by the following general formula (A), and examples of the styrylamine compound include compounds each represented by the following general formula (B). 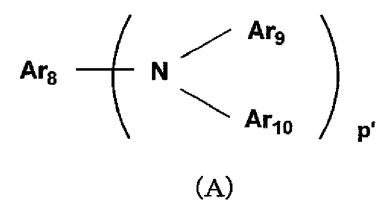 In the general formula (A), Ar₈ represents a group selected from phenyl, biphenyl, terphenyl, stilbene, and distyrylaryl groups, Ar₉ and Ar₁₀ each represent a hydrogen atom or an aromatic group having 6 to 20 carbon atoms and each of Ar₉ and Ar₁₀ may be substituted, p' represents an integer of 1 to 4, and at least one of Ar₉ and Ar₁₀ is more preferably substituted by a styryl group.
Here, preferable examples of the aromatic group having 6 to 20 carbon atoms include a phenyl group, a naphthyl group, an anthracenyl group, a phenanthryl group, and a terphenyl group. 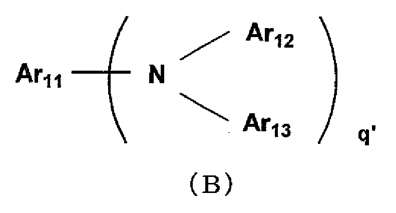 In the general formula (B), Ar₁₁ to Ar₁₃ each represent an aryl group which has 5 to 40 ring carbon atoms and which may be substituted, and q' represents an integer of 1 to 4.
Here, preferable examples of the aryl group having 5 to 40 ring atoms include phenyl, naphthyl, anthracenyl, phenanthryl, pyrenyl, coronyl, biphenyl, terphenyl, pyrrolyl, furanyl, thiophenyl, benzothiophenyl, oxadiazolyl, diphenylanthracenyl, indolyl, carbazolyl, pyridyl, benzoquinolyl, fluoranthenyl, acenaphthofluoranthenyl, and stilbene groups. It should be noted that the aryl group having 5 to 40 ring atoms may be additionally substituted by a substituent, and examples of a preferable substituent include an alkyl group having 1 to 6 carbon atoms (such as an ethyl group, a methyl group, an isopropyl group, an n-propyl group, an s-butyl group, a t-butyl group, a pentyl group, a hexyl group, a cyclopentyl group, or a cyclohexyl group), an alkoxy group having 1 to 6 carbon atoms (such as an ethoxy group, a methoxy group, an isopropoxy group, an n-propoxy group, an s-butoxy group, a t-butoxy group, a pentoxy group, a hexyloxy group, a cyclopentoxy group, or a cyclohexyloxy group), an aryl group having 5 to 40 ring atoms, an amino group substituted by an aryl group having 5 to 40 ring atoms, an ester group having an aryl group having 5 to 40 ring atoms, an ester group having an alkyl group having 1 to 6 carbon atoms, a cyano group, a nitro group, and a halogen atom (such as chlorine, bromine, or iodine).

A host material that can be used in a light emitting layer is preferably a compound represented by any one of the following formulae (i) to (ix).

An asymmetric anthracene represented by the following general formula (i):

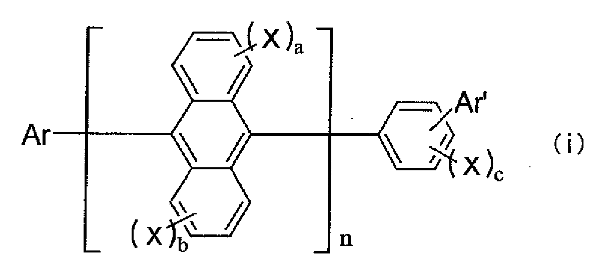

In the general formula: Ar represents a substituted or unsubstituted fused aromatic group having 10 to 50 ring atoms;
Ar' represents a substituted or unsubstituted aromatic group having 6 to 50 ring carbon atoms; X represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group, or a hydroxyl group.
a, b, and c each represent an integer of 0 to 4; and n represents an integer of 1 to 3. In addition, when n represents 2 or more, anthracene nuclei in [ ] may be identical to or different from each other.

An asymmetric monoanthracene derivative represented by the following general formula (ii):

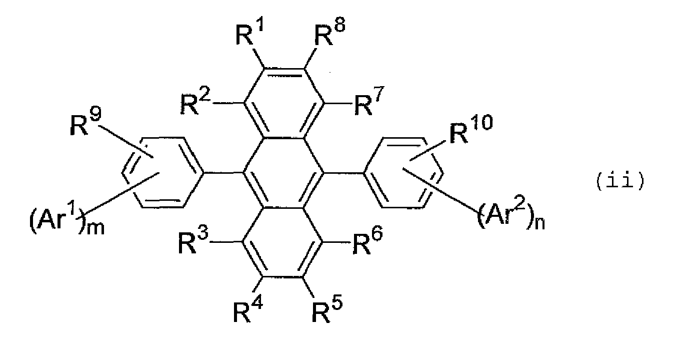

In the general formula: Ar¹ and Ar² each independently represent a substituted or unsubstituted aromatic ring group having 6 to 50 ring carbon atoms; m and n each represent an integer of 1 to 4, provided that Ar¹ and Ar² are not identical to each other when m = n = 1 and positions at which Ar¹ and Ar² are bound to a benzene ring are bilaterally symmetric, and m and n represent different integers when m or n represents an integer of 2 to 4; and
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxyl group.

An asymmetric pyrene derivative represented by the following general formula (iii):

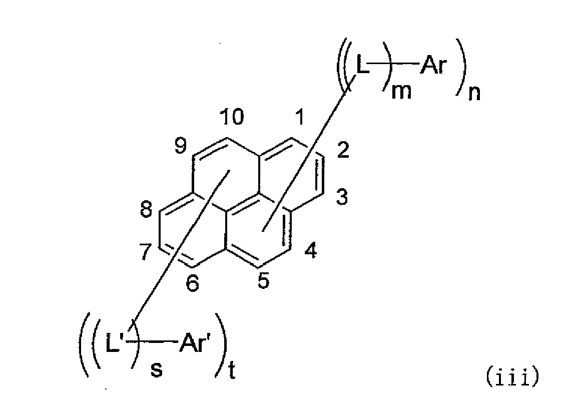

In the general formula: Ar and Ar' each represent a substituted or unsubstituted aromatic group having 6 to 50 ring carbon atoms;
L and L' each represent a substituted or unsubstituted phenylene group, a substituted or unsubstituted naphthalenylene group, a substituted or unsubstituted fluorenylene group, or a substituted or unsubstituted dibenzosilolylene group;
m represents an integer of 0 to 2. n represents an integer of 1 to 4. s represents an integer of 0 to 2. t represents an integer of 0 to 4; and
L or Ar binds to any one of 1- to 5-positions of pyrene, and L' or Ar' binds to any one of 6- to 10-positions of pyrene;
provided that Ar, Ar', L, and L' satisfy the following item
(1) or (2) when n + t represents an even number,

(1) Ar ≠ Ar' and/or L ≠ L' (where the symbol "≠" means that groups connected with the symbol have different structures)
(2) When Ar = Ar' and L = L',
   (2-1) m ≠ s and/or n ≠ t, or
   (2-2) when m = s and n = t,
      (2-2-1) L and L' (or pyrene) bind (or binds) to different binding positions on Ar and Ar', or
      (2-2-2) in the case where L and L' (or pyrene) bind (or binds) to the same binding positions on Ar and Ar',
   the case where the substitution positions of L and L', or of Ar and Ar' in pyrene are 1- and 6-positions, or 2- and 7-positions does not occur.

An asymmetric anthracene derivative represented by the following general formula (iv):

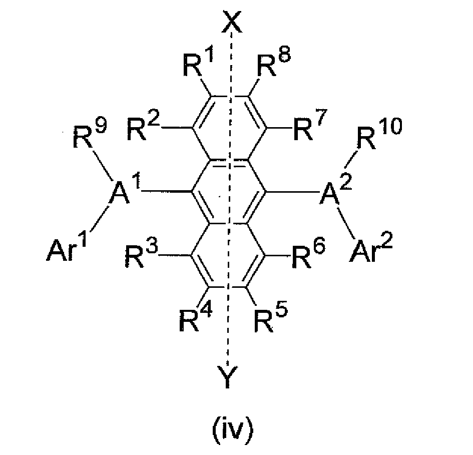

In the general formula: A¹ and A² each independently represent a substituted or unsubstituted fused aromatic ring group having 10 to 20 ring carbon atoms,;
Ar¹ and Ar² each independently represent a hydrogen atom, or a substituted or unsubstituted aromatic ring group having 6 to 50 ring carbon atoms;
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted orunsubstitutedaryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxyl group; and
the number of each of Ar¹, Ar², R⁹, and R¹⁰ may be two or more, and adjacent groups may form a saturated or unsaturated cyclic structure;
provided that the case where groups symmetric with respect to the X-Y axis shown on central anthracene in the general formula (1) bind to 9- and 10-positions of the anthracene does not occur.

An anthracene derivative represented by the following general formula (v):

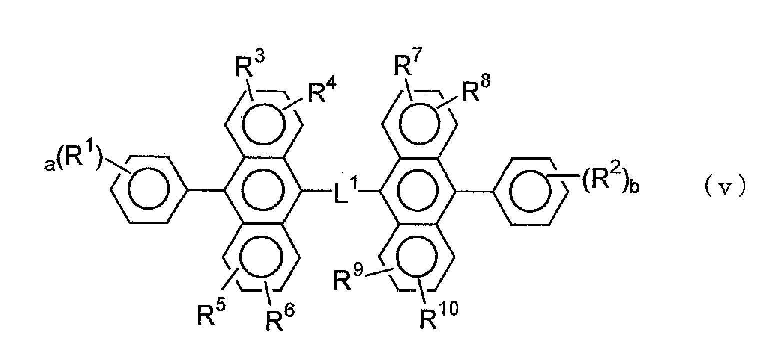

In the general formula: R¹ to R¹⁰ each independently represent a hydrogen atom, an alkyl group, a cycloalkyl group, an aryl group which may be substituted, an alkoxyl group, an aryloxy group, an alkylamino group, an alkenyl group, an arylamino group, or a heterocyclic group which may be substituted; a and b each represent an integer of 1 to 5, and, when a or b represents 2 or more, R¹'s or R²'s may be identical to or different from each other, or R¹'s or R²'s may be bonded to each other to form a ring; R³ and R⁴, R⁵ and R⁶, R⁷ and R⁸, or R⁹ and R¹⁰ may be bonded to each other to form a ring; and L¹ represents a single bond, -O-, -S-, -N(R)-where R represents an alkyl group or an aryl group which may be substituted, an alkylene group, or an arylene group.

An anthracene derivative represented by the following general formula (vi):

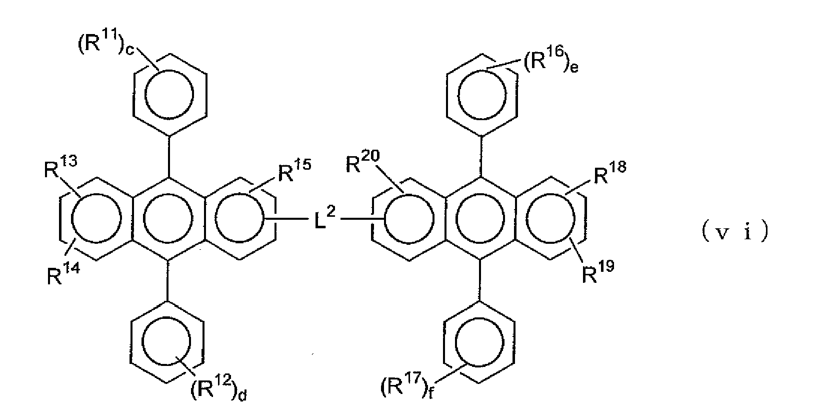

In the general formula: R¹¹ to R²⁰ each independently represent a hydrogen atom, an alkyl group, a cycloalkyl group, an aryl group, an alkoxyl group, an aryloxy group, an alkylamino group, an arylamino group, or a heterocyclic group which may be substituted; c, d, e, and f each represent an integer of 1 to 5, and, when any one of c, d, e, and f represents 2 or more, R¹¹'s, R¹²'s, R¹⁶'s, or R¹⁷'s may be identical to or different from each other, or R¹¹'s, R¹²'s, R¹⁶'s, or R¹⁷'s may be bonded to each other to form a ring; R¹³ and R¹⁴, or R¹⁸ and R¹⁹ may be bonded to each other to form a ring; and L² represents a single bond, -O-, -S-, -N (R) - where R represents an alkyl group or an aryl group which may be substituted, an alkylene group, or an arylene group.

A spirofluorene derivative represented by the following general formula (vii):

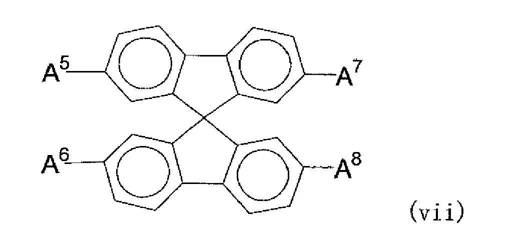

In the general formula, A⁵ to A⁸ each independently represent a substituted or unsubstituted biphenyl group, or a substituted or unsubstituted naphthyl group.

A fused ring-containing compound represented by the following general formula (viii):

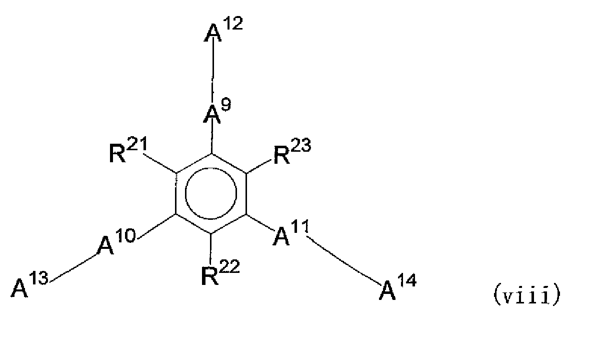

In the general formula: A⁹ to A¹⁴ each have the same meaning as that described above; R²¹ to R²³ each independently represent a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 carbon atoms, an aralkyloxy group having 7 to 18 carbon atoms, an arylamino group having 5 to 16 carbon atoms, a nitro group, a cyano group, an ester group having 1 to 6 carbon atoms, or a halogen atom; and at least one of A⁹ to A¹⁴ represents a group having three or more fused aromatic rings.

Afluorene compound represented by the following general formula (ix):

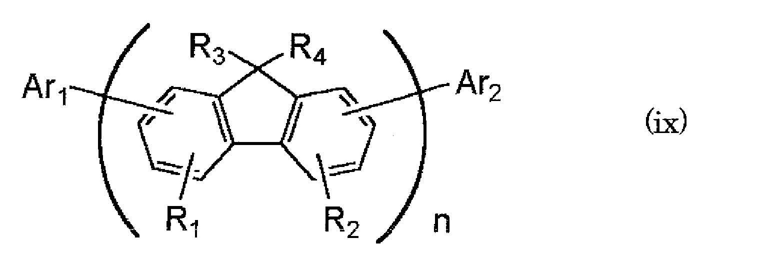

In the general formula: R₁ and R₂ each represent a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group, or a halogen atom; R₁'s or R₂'s bonded to different fluorene groups may be identical to or different from each other, and R₁ and R₂ bonded to the same fluorene group may be identical to or different from each other; R₃ and R₄ each represent a hydrogen atom, a substituted or unsubstituted alkyl group, a substituted or unsubstituted aralkyl group, a substituted or unsubstituted aryl group, or a substituted or unsubstituted heterocyclic group; R₃'s or R_{4'}s bonded to different fluorene groups may be identical to or different from each other, and R₃ and R₄ bonded to the same fluorene group may be identical to or different from each other; Ar₁ and Ar₂ each represent a substituted or unsubstituted fused polycyclic aromatic group having three or more benzene rings in total, or a substituted or unsubstituted fused polycyclic heterocyclic group that has three or more rings each of which is a benzene ring or a heterocyclic ring in total and that is bonded to a fluorene group by carbon, and Ar₁ and Ar₂ may be identical to or different from each other; and n represents an integer of 1 to 10.

Of the above-mentioned host materials, an anthracene derivative is preferable, a monoanthracene derivative is more preferable, and an asymmetric anthracene is particularly preferable.
In addition, a phosphorescent compound can also be used as a light emitting material for a dopant. A compound containing a carbazole ring as a host material is preferable as the phosphorescent compound. The dopant is preferably a compound capable of emitting light from a triplet exciton, and is not particularly limited as long as light is emitted from a triplet exciton, a metal complex containing at least one metal selected from the group consisting of Ir, Ru, Pd, Pt, Os, and Re is preferable.
A host composed of a compound containing a carbazole ring and suitable for phosphorescence is a compound having a function of causing a phosphorescent compound to emit light as a result of the occurrence of energy transfer from the excited state of the host to the phosphorescent compound. A host compound is not particularly limited as long as it is a compound capable of transferring exciton energy to a phosphorescent compound, and can be appropriately selected in accordance with a purpose. The host compound may have, for example, an arbitrary heterocyclic ring in addition to a carbazole ring.

Specific examples of such a host compound include a carbazole derivative, a triazole derivative, an oxazole derivative, an oxadiazole derivative, an imidazole derivative, a polyarylalkane derivative, a pyrazoline derivative, a pyrazolone derivative, a phenylene diamine derivative, an aryl amine derivative, an amino substituted chalcone derivative, a styrylanthracene derivative, a fluorenone derivative, a hydrazone derivative, a stilbene derivative, a silazane derivative, an aromatic tertiary amine compound, a styryl amine compound, an aromatic dimethylidene-based compound, a porphyrin-based compound, an anthraquinodimethane derivative, an anthrone derivative, a diphenylquinone derivative, a thiopyranedioxide derivative, a carbodiimide derivative, a fluorenilidene methane derivative, a distyryl pyrazine derivative, a heterocyclic tetracarboxylic anhydride such as naphthaleneperylene, a phthalocyanine derivative, various metal complex polysilane-based compounds typified by a metal complex of an 8-quinolinol derivative or a metal complex having metal phthalocyanine, benzooxazole, or benzothiazole as a ligand, polymer compounds such as a poly(N-vinylcarbazole) derivative, an aniline-based copolymer, a conductive high molecular weight oligomer such as a thiophene oligomer or polythiophene, a polythiophene derivative, a polyphenylene derivative, a polyphenylene vinylene derivative, and a polyfluorene derivative. One of the host materials may be used alone, or two or more of them may be used in combination.
Specific examples thereof include the compounds as described below.

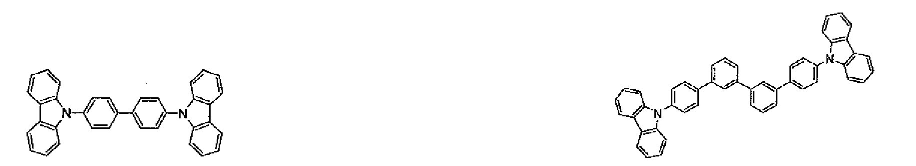

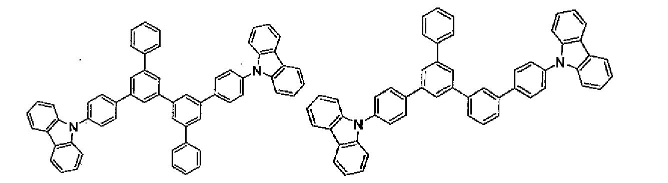

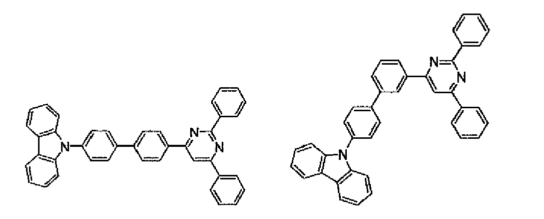

Aphosphorescent dopant is a compound capable of emitting light from a triplet exciton. The dopant, which is not particularly limited as long as light is emitted from a triplet exciton, is preferably a metal complex containing at least one metal selected from the group consisting of Ir, Ru, Pd, Pt, Os, and Re, and is preferably a porphyrin metal complex or an orthometalated metal complex. Aporphyrin platinum complex is preferable as the porphyrin metal complex. One kind of a phosphorescent compound may be used alone, or two or more kinds of phosphorescent compounds may be used in combination.
Any one of various ligands can be used for forming an orthometalated metal complex. Examples of a preferable ligand include a 2-phenylpyridine derivative, a 7,8-benzoquinoline derivative, a 2-(2-thienyl)pyridine derivative, a 2-(1-naphthyl)pyridine derivative, and a 2-phenylquinoline derivative. Each of those derivatives may have a substituent as required. A fluoride of any one of those derivatives, or one obtained by introducing a trifluoromethyl group into any one of those derivatives is a particularly preferable blue-based dopant. The metal complex may further include a ligand other than the above-mentioned ligands such as acetylacetonate or picnic acid as an auxiliary ligand.
The content of the phosphorescent dopant in the light emitting layer is not particularly limited, and can be appropriately selected in accordance with a purpose. The content is, for example, 0.1 to 70 mass%, and is preferably 1 to 30 mass%. When the content of the phosphorescent compound is less than 0.1 mass%, the intensity of emitted light is weak, and an effect of the incorporation of the compound is not sufficiently exerted. When the content exceeds 70 mass%, a phenomenon referred to as concentration quenching becomes remarkable, and device performance reduces.
In addition, the light emitting layer may contain a hole transporting material, an electron transporting material, or a polymer binder as required.
Further, the thickness of the light emitting layer is preferably 5 to 50 nm, more preferably 7 to 50 nm, or most preferably 10 to 50 nm. When the thickness is less than 5 nm, it becomes difficult to form the light emitting layer, so the adjustment of chromaticity may be difficult. When the thickness exceeds 50 nm, the voltage at which the device is driven may increase.

### (5) Hole injecting and transporting layer (hole transporting zone)

The hole injecting and transporting layer is a layer which helps injection of holes into the light emitting layer and transports the holes to the light emitting region. The layer exhibits a great mobility of holes and, in general, has an ionization energy as small as 5.5 eV or smaller. For such the hole injecting and transporting layer, a material which transports holes to the light emitting layer under an electric field of a smaller strength is preferable. A material which exhibits, for example, a mobility of holes of at least 10⁻⁴ cm²/V·sec under application of an electric field of 10⁴ to 10⁶ V/cm is preferable.
The material which can be used for forming the hole injecting and transporting layer is not particularly limited as long as the material has a preferable property described above. The material can be arbitrarily selected from materials which are conventionally used as the charge transporting material of holes in photoconductive materials and known materials which are used for the hole injecting and transporting layer in organic EL devices.

Specific examples include: a triazole derivative (see, for example, US 3,112,197); an oxadiazole derivative (see, for example, US 3,189,447); an imidazole derivative (see, for example, JP-B-37-16096); a polyarylalkane derivative (see, for example, US 3, 615, 402, US 3,820,989, US 3, 542, 544, JP-B-45-555, JP-B-51-10983, JP-A-51-93224, JP-A-55-17105, JP-A-56-4148, JP-A-55-108667, JP-A-55-156953, and JP-A-56-36656); a pyrazoline derivative and a pyrazolone derivative (see, for example, US 3,180,729, US 4,278,746, JP-A-55-88064, JP-A-55-88065, JP-A-49-105537, JP-A-55-51086, JP-A-56-80051, JP-A-56-88141, JP-A-57-45545, JP-A-54-112637, and JP-A-55-74546); a phenylenediamine derivative (see, for example, US 3,615,404, JP-B-51-10105, JP-B-46-3712, JP-B-47-25336, JP-A-54-53435, JP-A-54-110536, and JP-A-54-119925); an arylamine derivative (see, for example, US 3,567,450, US 3,180,703, US 3, 240, 597, US 3,658,520, US 4,232,103, US 4, 175, 961, US 4, 012, 376, JP-B-49-35702, JP-B-39-27577, JP-A-55-144250, JP-A-56-119132, JP-A-56-22437, and DE 1,110,518); an amino-substituted chalcone derivative (see, for example, US 3, 526, 501) ; an oxazole derivative (those disclosed in US 3,257,203); a styrylanthracene derivative (see, for example, JP-A-55-46234); a fluorenone derivative (see, for example, JP-A-54-110837); a hydrazone derivative (see, for example, US 3, 717,462, JP-A-54-59143, JP-A-55-52063, JP-A-55-52064, JP-A-55-46760, JP-A-55-85495, JP-A-57-11350, JP-A-57-148749, and JP-A-2-311591); a stilbene derivative (see, for example, JP-A-61-210363, JP-A-61-228451, JP-A-61-14642, JP-A-61-72255, JP-A-62-47646, JP-A-62-36674, JP-A-62-10652, JP-A-62-30255, JP-A-60-93445, JP-A-60-94462, JP-A-60-174749, and JP-A-60-175052); a silazane derivative (US 4,950,950); a polysilane-based copolymer (JP-A-2-204996); an aniline-based copolymer (JP-A-2-282263); and a conductive high molecular weight oligomer (particularly a thiophene oligomer) disclosed in JP-A-1-211399.

In addition to the above-mentioned materials which can be used as the material for the hole injecting and transporting layer, a porphyrin compound (those disclosed in, for example, JP-A-63-2956965); an aromatic tertiary amine compound and a styrylamine compound (see, for example, US 4,127,412, JP-A-53-27033, JP-A-54-58445, JP-A-54-149634, JP-A-54-64299, JP-A-55-79450, JP-A-55-144250, JP-A-56-119132, JP-A-61-295558, JP-A-61-98353, and JP-A-63-295695) are preferable, and aromatic tertiary amines are particularly preferable.

A compound represented by the following general formula (x) is a preferable hole injecting/transporting material that can be used in the hole injecting/transporting layer. 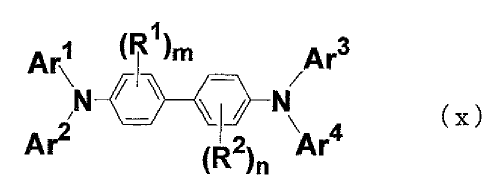 Ar¹ to Ar⁴ each independently represent a substituted or unsubstituted aryl group having 6 to 50 ring carbon atoms, R¹ and R² each independently represent a hydrogen atom, a substituted or unsubstituted aryl group having 6 to 50 ring carbon atoms, or an alkyl group having 1 to 50 carbon atoms, and m and n each represent an integer of 0 to 4.
Preferable examples of the aryl group having 6 to 50 ring carbon atoms include phenyl, naphthyl, biphenyl, terphenyl, and phenanthryl groups. It should be noted that the aryl group having 6 to 50 ring carbon atoms may be additionally substituted by a substituent, and examples of a preferable substituent include an alkyl group having 1 to 6 carbon atoms (such as a methyl group, an ethyl group, an isopropyl group, an n-propyl group, an s-butyl group, a t-butyl group, a pentyl group, a hexyl group, a cyclopentyl group, or a cyclohexyl group) and an amino group substituted by an aryl group having 6 to 50 ring carbon atoms.

In addition, a substance having two fused aromatic rings in any one of its molecules described in US Patent No. 5,061,569 such as 4,4'-bis(N-(1-naphthyl)-N-phenylamino)biphenyl (hereinafter abbreviated as "NPD"), and 4,4',4''-tris(N-(3-methylphenyl)-N-phenylamino)triphenylamine (hereinafter abbreviated as "MTDATA") described in Japanese Patent Application Laid-Open No. Hei 4-308688 in which three triphenylamine units are linked with one another in a starburst fashion can be given as examples.
Further, an inorganic compound such as p-type Si or p-type SiC as well as the above-mentioned aromatic dimethylidine-based compound shown as a material for the light emitting layer can also be used as a material for the hole injecting/transporting layer.

The hole injecting and transporting layer can be formed by forming a thin layer from the compound in accordance with a known process such as the vacuum vapor deposition process, the spin coating process, the casting process, and the LB process. The thickness of the hole injecting and transporting layer is not particularly limited. In general, the thickness is 5 nm to 5 µm. The hole injecting and transporting layer may be constituted of a single layer containing one or more materials described above or may be a laminate constituted of hole injecting and transporting layers containing materials different from the materials of the hole injecting and transporting layer described above as long as the compound is incorporated in the hole injecting and transporting zone.
Further, an organic semiconductor layer may be disposed as a layer for helping the injection of holes or electrons into the light emitting layer. As the organic semiconductor layer, a layer having a conductivity of 10⁻¹⁰ S/cm or greater is preferable. As the material for the organic semiconductor layer, oligomers containing thiophene, conductive oligomers such as oligomers containing arylamine and conductive dendrimers such as dendrimers containing arylamine which are disclosed in JP-A-08-193191, a tetracyanodamethane derivative, hexacyanohexaazatriphenylene (JP 03614405) and the like, can be used.

### (6) Electron injecting/transporting layer (electron transporting zone)

The electron injecting/transporting layer is a layer which: aids the injection of an electron into the light emitting layer; and transports the electron to a light emitting region. The layer has a large electron mobility, and typically shows an electron affinity as large as 2.5 eV or more. A material that transports an electron to the light emitting layer at additionally weak electric field intensity is preferably used in such electron injecting/transporting layer, and, furthermore, an electron mobility at the time of the application of, for example, an electric field of 10⁴ to 10⁶ V/cm is preferably at least 10⁻⁶ cm²/V·sec.
When the nitrogen-containing heterocyclic derivative of the present invention is used in an electron transporting zone, the electron injecting/transporting layer may be formed only of the nitrogen-containing heterocyclic derivative of the present invention, or may be formed of a mixture containing the derivative and any other material.
The material to be mixed with the nitrogen-containing heterocyclic derivative of the present invention to form the electron injecting/transporting layer is not particularly limited as long as the material has the above-mentioned preferable nature, and a material to be used can be arbitrarily chosen from a material that has been conventionally used as a charge transporting material for an electron in a photoconductive material and a known material to be used in the electron injecting/transporting layer of an organic EL device.
In addition, the adhesion improving layer is a layer composed of a material that adheres particularly well to a cathode in the electron injecting layers. In the organic EL device of the present invention, the above compound of the present invention is preferably used in an electron injecting/transporting layer or an adhesion improving layer.

A preferable embodiment of the organic EL device of the present invention includes an element including a reducing dopant in the interfacial region of the region of electron transport or the cathode and the organic thin film layer. In the present invention, the organic El device containing the reducing dopant with the compound of the present invention is preferable. The reducing dopant is defined as a substance which can reduce a compound having the electron-transporting property. Various compounds can be used as the reducing dopant as long as the compounds have a certain reductive property. For example, at least one substance selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals, and organic complexes of rare earth metals can be preferably used.
More specifically, examples of the reducing dopant include substances having a work function of 2.9 eV or smaller, specific examples of which include at least one alkali metal selected from the group consisting of Na (the work function: 2.36 eV), K (the work function: 2.28 eV), Rb (the work function: 2.16 eV), and Cs (the work function: 1.95 eV) and at least one alkaline earth metal selected from the group consisting of Ca (the work function: 2.9 eV) , Sr (the work function: 2. 0 to 2. 5 eV), and Ba (the work function: 2.52 eV). Among the above-mentioned substances, at least one alkali metal selected from the group consisting of K, Rb, and Cs is more preferable, Rb and Cs are still more preferable, and Cs is most preferable as the reducing dopant. Those alkali metals have great reducing ability, and the luminance of the emitted light and the life time of the organic EL device can be increased by addition of a relatively small amount of the alkali metal into the electron injecting zone. As the reducing dopant having a work function of 2.9 eV or smaller, combinations of two or more alkali metals thereof are also preferable. Combinations having Cs such as the combinations of Cs and Na, Cs and K, Cs and Rb, and Cs, Na, and K are more preferable. The reducing ability can be efficiently exhibited by the combination having Cs. The luminance of emitted light and the life time of the organic EL device can be increased by adding the combination having Cs into the electron injecting zone.

The present invention may further include an electron injecting layer which is composed of an insulating material or a semiconductor and disposed between the cathode and the organic layer.
At this time, leak of electric current can be effectively prevented and the electron injecting property can be improved. As the insulating material, at least one metal compound selected from the group consisting of alkali metal chalcogenides, alkaline earth metal chalcogenides, alkali metal halides, and alkaline earth metal halides is preferable. It is preferable that the electron injecting layer be composed of the above-mentioned substance such as the alkali metal chalcogenide since the electron injecting property can be further improved. Preferable examples of the alkali metal chalcogenide include Li₂O, K₂O, Na₂S, Na₂Se, and Na₂O. To be specific, preferable examples of the alkaline earth metal chalcogenide include CaO, BaO, SrO, BeO, BaS, and CaSe. Preferable examples of the alkali metal halide include LiF, NaF, KF, LiCl, KCl, and NaCl. Preferable examples of the alkaline earth metal halide include fluorides such as CaF₂, BaF₂, SrF₂, MgF₂, and Be_{F}2 and halides other than the fluorides.
Examples of the semiconductor composing the electron-transporting layer include oxides, nitrides, and oxide nitrides of at least one element selected from Ba, Ca, Sr, Yb, Al, Ga, In, Li, Na, Cd, Mg, Si, Ta, Sb, and Zn used alone or in combination of two or more. It is preferable that the inorganic compound composing the electron-transporting layer form a crystallite or amorphous insulating thin film. When the electron injecting layer is composed of the insulating thin film described above, a more uniform thin film can be formed, and defects of pixels such as dark spots can be decreased. Examples of the inorganic compound include alkali metal chalcogenides, alkaline earth metal chalcogenides, alkali metal halides, and alkaline earth metal halides which are described above.

### (7) Cathode

As the cathode, a material such as ametal, an alloy, a conductive compound, or a mixture of those materials which has a small work function (4 eV or smaller) is used because the cathode is used for injecting electrons to the electron injecting and transporting layer or the light emitting layer. Specific examples of the electrode material include sodium, sodium-potassium alloys, magnesium, lithium, magnesium-silver alloys, aluminum/aluminum oxide, aluminum-lithium alloys, indium, and rare earth metals.
The cathode can be prepared by forming a thin film of the electrode material described above in accordance with a process such as the vapor deposition process and the sputtering process.
When the light emitted from the light emitting layer is obtained through the cathode, it is preferable that the cathode have a transmittance of the emitted light greater than 10%.
It is also preferable that the sheet resistivity of the cathode be several hundred Ω/□ or smaller. The thickness of the cathode is generally in the range of 10 nm to 1 µm and preferably in the range of 50 to 200 nm.

### (8) Insulating layer

Defects in pixels tend to be formed in organic EL device due to leak and short circuit because an electric field is applied to ultra-thin films. To prevent the formation of the defects, a layer of a thin film having an insulatingpropertymaypreferablybe inserted between the pair of electrodes.
Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. Mixtures and laminates of the above-mentioned materials may also be used.

### (9) Method of producing the organic EL device

The anode and the light emitting layer, and, when necessary, the hole injecting and the transporting layer and the electron injecting and transporting layer are formed by the illustrated materials and the illustrated process, and further the cathode is formed, to thereby prepare the organic EL device of the present invention. The organic EL device may also be prepared in the order reverse to that described above, i.e., from the cathode to the anode.
Hereinafter, an description will be made of a manufacturing example of an organic EL device having a construction in which an anode, a hole injecting layer, a light emitting layer, an electron injecting layer, and a cathode are formed successively on a substrate transmitting light.
On a suitable transparent substrate, a thin film made of a material for the anode is formed the vapor deposition process or the sputtering process so as to have the thickness of 1 µm or smaller, and preferably in the range of 10 to 200 nm, thereby forming the anode. Then, a hole injecting layer is formed on the anode. The hole injecting layer can be formed by the vacuum vapor deposition process, the spin coating process, the casting process, or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions be suitably selected in the following ranges: the temperature of the source of the deposition: 50 to 450°C; the vacuum: 10⁻⁷ to 10⁻³ Torr; the rate of deposition: 0.01 to 50 nm/sec; the temperature of the substrate: -50 to 300°C and the thickness of the film: 5 nm to 5 µm; although the conditions of the vacuum vapor deposition are different depending on the compound to be used (i.e., the material for the hole injecting layer) and the crystal structure and the recombination structure of the target hole injecting layer.

Then, the formation of the light emitting layer including forming the light emitting layer on the hole injecting layer may be performed by using a desired organic light emitting material in accordance with a process such as the vacuum vapor deposition process, the sputtering process, the spin coating process, or the casting process, thereby making the organic light emitting material into a thin film. The vacuum vapor deposition process is preferable since a uniform film can be easily obtained and the possibility of formation of pin holes is small. When the light emitting layer is formed in accordance with the vacuum vapor deposition process, in general, the conditions of the vacuum vapor deposition process can be selected in the same ranges as those described for the vacuum vapor deposition of the hole injecting layer, although the conditions are different depending on the compound to be used.
Next, an electron injecting layer is formed on the light emitting layer. Similarly to the hole injecting layer and the light emitting layer, it is preferable that the electron injecting layer be formed in accordance with the vacuum vapor deposition process since a uniform film needs to be obtained. The conditions of the vacuum vapor deposition can be selected in the same ranges as those described for the vacuum vapor deposition of the hole injecting layer and the light emitting layer.
When the vapor deposition process is used, the nitrogen-containing heterocyclic derivative of the present invention can be deposited by vapor in combination with other materials, although the situation may be different depending on which layer in the light emitting zone or in the hole transporting zone includes the derivative. When the spin coating process is used, the derivative can be incorporated by using a mixture of the derivative with other materials.
Finally, a cathode is laminated on the electron injecting layer, and an organic EL device can be obtained.
The cathode is made of a metal and can be formed in accordance with the vacuum vapor deposition process or the sputtering process. It is preferable that the vacuum vapor deposition process is used in order to prevent formation of damages on the lower organic layers during the formation of the film.
In the above-mentioned preparation of the organic EL device, it is preferable that the above-mentioned layers from the anode to the cathode are formed successively while the preparation system is kept in a vacuum after being evacuated once.

The method of forming the layers in the organic EL device of the present invention is not particularly limited. A conventionally known process such as the vacuum vapor deposition process or the spin coating process can be used. The organic thin film layer which is used in the organic EL device of the present invention and includes the compound represented by general formula (1) described above can be formed in accordance with a known process such as the vacuum vapor deposition process or the molecular beam epitaxy process (the MBE process) or, a preparation of a solution by dissolving the compounds into a solvent followed by a coating process such as the dipping process, the spin coating process, the casting process, the bar coating process, or the roll coating process.
The thickness of each layer in the organic thin film layer in the organic EL device of the present invention is not particularly limited. In general, an excessively thin layer tends to have defects such as pin holes, and an excessively thick layer requires a high applied voltage to decrease the efficiency. Therefore, a thickness in the range of several nanometers to 1 µm is preferable.
The emission of light can be observed when a direct voltage of 5 to 40 V is applied to the organic EL device in the condition that the polarity of the anode is positive (+) and the polarity of the cathode is negative (-). When the polarity is reversed and a voltage is applied, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### EXAMPLES

In the synthesis examples of the present invention, first, Intermediates 1 to 4 shown below were synthesized from corresponding benzil, pyridil, 4-bromoaniline, 4-bromobenzaldehyde, aniline, and benzaldehyde by a method by K. Buttke et al. (Journal fur praktische Chemie Chemiker-Zeitung, (339), 1997, 721-728).

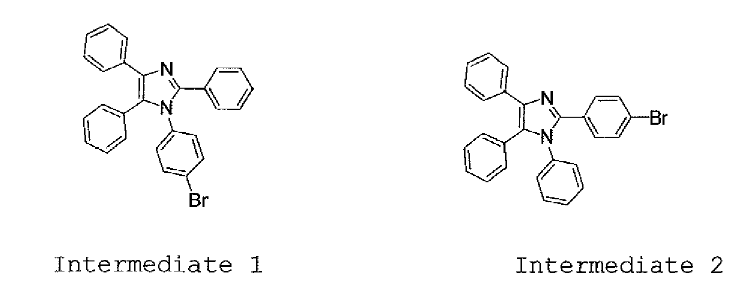

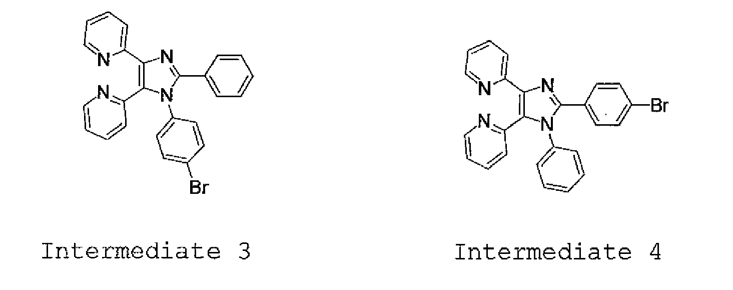

### Synthesis Example 1: Synthesis of Compound (1)

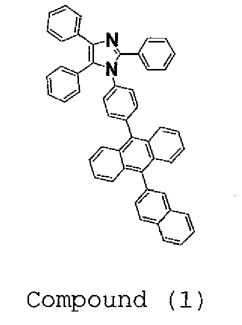

In a stream of argon, 3.0 g (6.6 mmol) of Intermediate 1, 2.5 g (8.9 mmol) of 10-naphthalen-2-yl-anthracene-9-boronic acid, 0.16 g (0.14 mmol) of tetrakis(triphenylphosphine)palladium(0), 30 mL of 1,2-dimethoxyethane, and 11 mL (22 mmol) of a 2-M aqueous solution of sodium carbonate were added to a 300-mL three-necked flask, and the whole was refluxed under heat for 8 hours. After the completion of the reaction, dichloromethane was added, and the resultant was sufficiently washed with water and dried with magnesium sulfate. After that, the solvent was removed by distillation with a rotary evaporator. The resultant coarse crystal was purified by means of silica gel column chromatography (developing solvent: chloroform), and was then washed with 100 mL of methanol, whereby 2.6 g of a pale yellow powder were obtained. The powder was identified as Compound (1) by field desorption mass spectrometry (FD-MS) (60% yield).

### Synthesis Example 2: Synthesis of Compound (2)

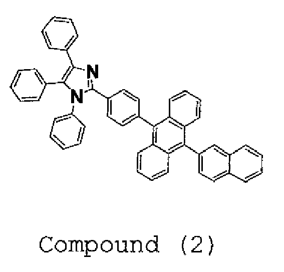

3.0 g of Compound (2) as a pale yellow powder were obtained (69% yield) by performing the same operation as that of the synthesis of Compound (1) except that Intermediate 2 was used instead of Intermediate 1. The powder was identified as Compound (2) by field desorption mass spectrometry (FD-MS).

### Synthesis Example 3: Synthesis of Compound (3)

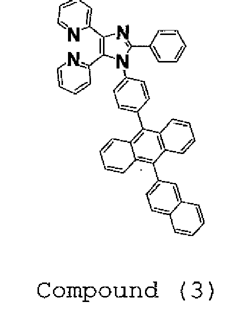

2.2 g of Compound (3) as a pale yellow powder were obtained (51% yield) by performing the same operation as that of the synthesis of Compound (1) except that Intermediate 3 was used instead of Intermediate 1. The powder was identified as Compound (3) by field desorption mass spectrometry (FD-MS).

### Synthesis Example 4: Synthesis of Compound (4)

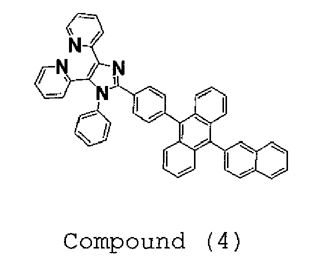

2.0 g of Compound (4) as a pale yellow powder were obtained (46% yield) by performing the same operation as that of the synthesis of Compound (1) except that Intermediate 2 was used instead of Intermediate 1. The powder was identified as Compound (4) by field desorption mass spectrometry (FD-MS).

### Example 1 (production of organic EL device using the compound of the present invention in its electron transporting layer)

A glass substrate measuring 25 mm wide by 75 mm long by 1.1 mm thick and provided with an ITO transparent electrode (anode) (manufactured by GEOMATEC Co., Ltd.) was subjected to ultrasonic cleaning in isopropyl alcohol for 5 minutes. After that, the resultant was subjected to UV ozone cleaning for 30 minutes. The glass substrate provided with a transparent electrode line after the cleaning was mounted on a substrate holder of a vacuum deposition device, and, first, an N,N'-bis(N,N'-diphenyl-4-aminophenyl)-N,N-diphenyl-4,4'-diamin 0-1,1'-biphenyl film (hereinafter abbreviated as "TPD232 film") having a thickness of 60 nm was formed on the surface on the side where the transparent electrode line was formed so as to cover the transparent electrode. The TPD232 film functions as a hole injecting layer. Subsequent to the formation of the TPD232 film, a 4,4'-bis[N-(1-naphthyl)-N-phenylamino]biphenyl film (hereinafter abbreviated as "NPD film") having a thickness of 20 nm was formed on the TPD232 film. The NPD film functions as a hole transporting layer.
Further, Anthracene Derivative A1 and Styrylamine Derivative S1 represented by the following formulae were formed into a film having a thickness of 40 nm at a thickness ratio of 40 : 2 on the NPD film, whereby a bluish light emitting layer was obtained.

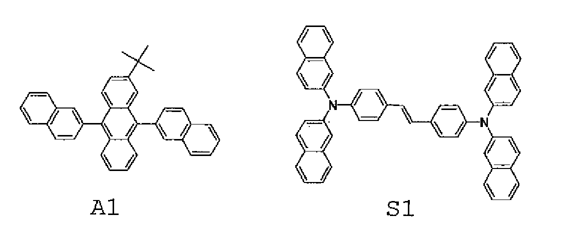

Compound (1) was formed by vapor deposition into a film having a thickness of 20 nm to serve as an electron transporting layer on the film. After that, LiF was formed into a film having a thickness of 1 nm. Metal Al was vapor-deposited onto the LiF film to form a metal cathode having a thickness of 150 nm, thus an organic EL light emitting device was formed.

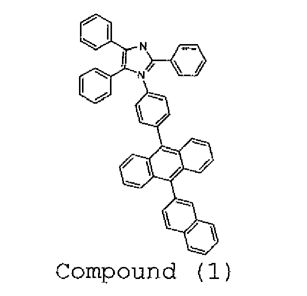

### Example 2

An organic EL device was produced in the same manner as in Example 1 except that Compound (2) was used instead of Compound (1).

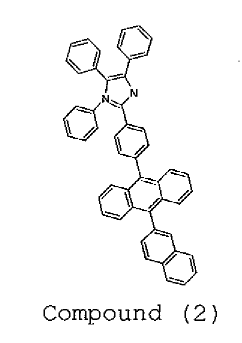

### Example 3

An organic EL device was produced in the same manner as in Example 1 except that Compound (3) was used instead of Compound (1).

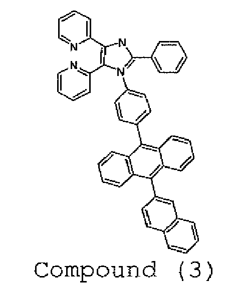

### Example 4

An organic EL device was produced in the same manner as in Example 1 except that Compound (4) was used instead of Compound (1).

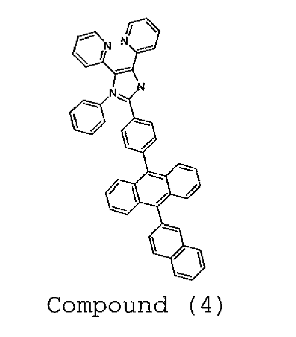

### Comparative Example 1

An organic EL device was produced in the same manner as in Example 1 except that Compound A shown below described in International Patent WO 2004/080975 A1 was used instead of Compound (1).

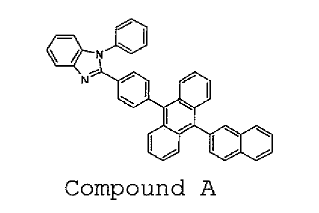

### Comparative Example 2

An organic EL device was produced in the same manner as in Example 1 except that Compound B shown below described in International Patent WO 2004/080975 A1 was used instead of Compound (1).

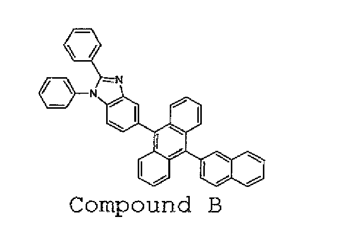

### Comparative Example 3

An organic EL device was produced in the same manner as in Example 1 except that Alq (aluminum complex of 8-hydroxyquinoline) was used instead of Compound (1).

### (Evaluation of organic EL device)

The emission luminance, luminous efficiency, and chromaticity of each of the organic EL devices obtained in Examples 1 to 4 and Comparative Examples 1 to 3 described above were measured while a direct voltage described in Table 1 below was applied, and the luminescent color of each of the devices was observed. Table 1 shows the results.

**Table 1**

| | Compound of electron injecting layer | Voltage (v) | Current density (mA/m²) | Emission luminanc e (cd/m²) | luminous efficien cy (cd/A) | Lumine scent color |
|---|---|---|---|---|---|---|
| Example 1 | Compound (1) | 4.7 | 10.0 | 790.5 | 7.91 | Blue |
| Example 2 | Compound (2) | 4.5 | 10.0 | 794.7 | 7.95 | Blue |
| Example 3 | Compound (3) | 4.8 | 10.0 | 781.9 | 7.82 | Blue |
| Example 4 | Compound (4) | 4.8 | 10.0 | 784.2 | 7.84 | Blue |
| Comparative Example 1 | Compound A | 6.1 | 10.0 | 622.9 | 6.23 | Blue |
| Comparative Example 2 | Compound B | 5.3 | 10.0 | 740.0 | 7.40 | Blue |
| Comparative Example 3 | Alq | 6.2 | 10.0 | 480.3 | 4.80 | Blue |

As can be seen from the results shown in Table 1 above, a device having extremely high emission luminance and extremely high luminous efficiency can be produced by using any one of the above compounds in an electron injecting layer.

### INDUSTRIAL APPLICABILITY

As described above in detail, the use of the nitrogen-containing heterocyclic derivative of the present invention in at least one layer of the organic thin film layer of an organic EL device can achieve high luminous efficiency even at a low voltage, and high luminous efficiency caused by excellent electron transporting property. Therefore, the organic EL device of the present invention is extremely useful as, for example, a light source for any one of various electronic instruments.

## Claims

1. A nitrogen-containing heterocyclic derivative represented by the following general formula (1): 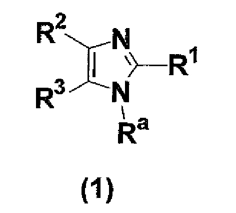 in the general formula (1):
R¹ to R³ each independently represent a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substitutedor unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted by a substituted or unsubstituted aryl group having 6 to 60 ring carbon atoms, a halogen atom, a cyano group, a nitro group, a hydroxyl group, or a carboxyl group;
R^{a} represents a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, or a substituted or unsubstituted aralkyl group having 6 to 50 ring atoms; and
at least one of R¹ to R³ and R^{a} represents a substituent represented by the following general formula (2): 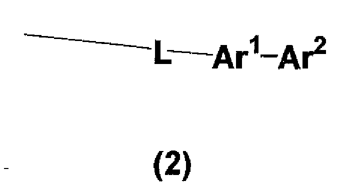
where:
L represents a single bond, a substituted or unsubstituted arylene group having 6 to 60 ring carbon atoms, a substituted or unsubstituted heteroarylene group having 5 to 60 ring atoms, or a substituted or unsubstituted fluorenylene group;
Ar¹ represents a substituted or unsubstituted arylene group having 6 to 60 ring carbon atoms, a substituted or unsubstituted heteroarylene group having 5 to 60 ring atoms, or a substituted or unsubstituted fluorenylene group; and
Ar² represents a hydrogen atom, a substituted or unsubstituted aryl group having 6 to 60 ring carbon atoms, a substituted or unsubstituted heteroaryl group having 5 to 60 ring atoms, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted by a substituted or unsubstituted aryl group having 6 to 50 ring carbon atoms, a halogen atom, a cyano group, a nitro group, a hydroxyl group, or a carboxyl group.

2. A nitrogen-containing heterocyclic derivative according to claim 1, wherein the compound represented by the general formula (1) comprises a compound represented by the following general formula (1-a), (1-b), or (1-c): 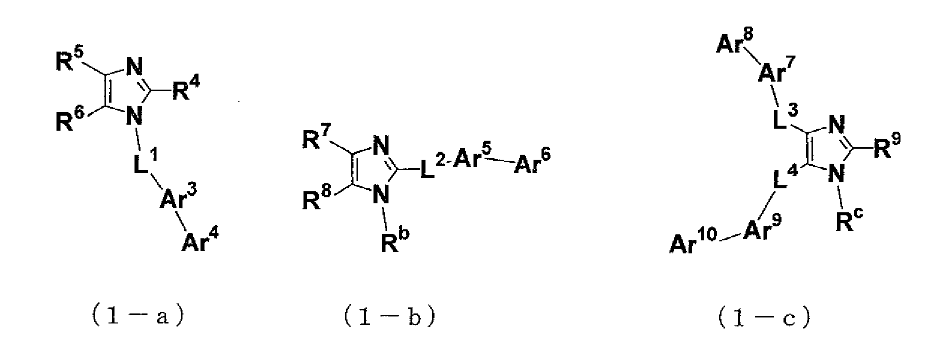 in the general formula, R⁴ to R⁹ each independently have the same meaning as that of any one of R¹ to R³ in the general formula (1), Ar³, Ar⁵, Ar⁷, and Ar⁹ each independently have the same meaning as that of Ar¹ in the general formula (2), Ar⁴, Ar⁶, Ar⁸, and Ar¹⁰ each independently have the same meaning as that of Ar² in the general formula (2), R^{b} and R^{c} each independently have the same meaning as that of R^{a} in the general formula (1), and L¹, L², L³, and L⁴ each independently have the same meaning as that of L in the general formula (2).

3. A nitrogen-containing heterocyclic derivative according to claim 1, wherein the compound represented by the general formula (1) comprises a compound represented by the following general formula (1-d) or (1-e): 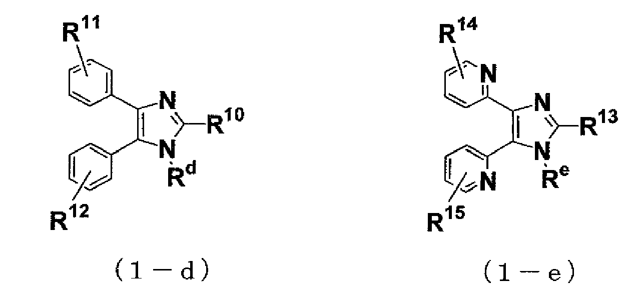 where:
R^{d} and R^{e} each independently have the same meaning as that of R^{a} in the general formula (1); and
at least one of R¹⁰ and R^{d} in the general formula (1-d) represents a substituent represented by the general formula (2), and at least one of R¹³ and R^{e} in the general formula (1-e) represents a substituent represented by the general formula (2).

4. A nitrogen-containing heterocyclic derivative according to any one of claims 1 to 3, wherein the nitrogen-containing heterocyclic derivative comprises a material for an organic electroluminescence device.

5. A nitrogen-containing heterocyclic derivative according to any one of claims 1 to 3, wherein the nitrogen-containing heterocyclic derivative comprises an electron injecting material for an organic electroluminescence device or an electron transporting material for an organic electroluminescence device.

6. A nitrogen-containing heterocyclic derivative according to any one of claims 1 to 3, wherein the nitrogen-containing heterocyclic derivative comprises a light emitting material for an organic electroluminescence device.

7. An organic electroluminescence device comprising an organic thin film layer composed of one or a plurality of layers including at least a light emitting layer and interposed between a cathode and an anode, wherein at least one layer of the organic thin film layer contains the nitrogen-containing heterocyclic derivative according to any one of claims 1 to 3 alone or as a component of a mixture.

8. An organic electroluminescence device according to claim 7, wherein the organic thin film layer has an electron injecting layer or an electron transporting layer, and the electron injecting layer or the electron transporting layer contains the nitrogen-containing heterocyclic derivative according to any one of claims 1 to 3 alone or as a component of a mixture.

9. An organic electroluminescence device according to claim 7, wherein the light emitting layer contains the nitrogen-containing heterocyclic derivative according to any one of claims 1 to 3 alone or as a component of a mixture.

10. An organic electroluminescence device according to claim 7, wherein the electron injecting layer or the electron transporting layer containing the nitrogen-containing heterocyclic derivative contains a reducing dopant.

11. An organic electroluminescence device according to claim 10, wherein the reducing dopant comprises at least one kind of a substance selected from the group consisting of an alkali metal, an alkaline earth metal, a rare earth metal, an alkali metal oxide, an alkali metal halide, an alkaline earth metal oxide, an alkaline earth metal halide, a rare earth metal oxide, a rare earth metal halide, an organic complex of an alkali metal, an organic complex of an alkaline earth metal, and an organic complex of a rare earth metal.
